# EUROPEAN PATENT SPECIFICATION

(11) **EP 4 272 701 B1**
(45) Date of publication and mention of the grant of the patent: **27.08.2025**
(21) Application number: 21913011.9
(22) Date of filing: 12.07.2021
(51) Int. Cl.: A61F 2/24

(54) **SYSTEM FOR CLAMPING TISSUE**
SYSTEM ZUM KLEMMEN VON GEWEBE
SYSTÈME DE PINCEMENT TISSULAIRE

(30) Priority: 30.12.2020 CN 202011622698
(43) Date of publication of application: 08.11.2023
(73) Proprietor: Sierra Valve LLC, Wilmington, DE 19801 (US)
(72) Inventor: TAN, Jian Fong, Suzhou, Jiangsu 215000 (CN); WANG, Kai, Suzhou, Jiangsu 215000 (CN); ZHANG, Yi, Suzhou, Jiangsu 215000 (CN)
(74) Representative: Huang, Liwei
(86) International application number: PCT/CN2021/105801
(87) International publication number: WO 2022/142258

(56) References cited:
- EP-A1- 3 643 255
- CN-A- 107 106 176
- CN-A- 110 536 656
- CN-A- 111 449 696
- CN-A- 112 656 546
- CN-U- 210 811 305
- CN-U- 211 560 531
- CN-U- 211 560 531
- US-A1- 2019 261 997

## Description

### TECHNICAL FIELD

The present disclosure relates to a medical device, and in particular to a system for clamping a tissue.

### BACKGROUND

With medical advances, conventional high-risk surgeries are being gradually replaced by minimally invasive catheter procedures. At present, minimally invasive techniques mainly developed and applied in markets include indirect annuloplasty, direct annuloplasty, edge-to-edge repair, and chordae tendineae repair.

The edge-to-edge repair gradually becomes mature in clinical practices of surgical treatment for mitral valve regurgitation and has a desirable treatment effect.

Valve clamp devices developed based on the technical principle of the surgical edge-to-edge suture of the valve are currently most recognized due to high safety, simple technical principle, and high feasibility.

EP 3643255 A1 describes a clip treatment tool for an endoscope, which is used, for example, to close a wound and to stop bleeding in a living body. A clip treatment tool includes a clip unit and a treatment tool body. The clip unit includes a fixing pin, and a clip body, which has a first clip piece and a second clip piece. The arm portions of the two clip pieces open in accordance with movement of the clip body from the proximal end side toward the distal end side, and the arm portions of the two clip pieces close in accordance with movement of the clip body from the distal end side toward the proximal end side.

According to the prior art, to use a valve clamp device, it needs a delivery assembly for delivering the valve clamp device to a target location on the heart. Then, the valve clamp device may be fixed there and stay in the heart as an implant, while the delivery assembly may be relatively separated from the valve clamping device by means of a clutch mechanism. However, existing clamps are restricted in length to some extent, because they need to pass through narrow channels and make a turn, which limits a capturing distance. Moreover, existing connecting rod mechanisms for driving the clamp also limit a turning angle of the clamp, which makes it more difficult to capture a valve leaflet. To sum up, it is desired to improve both the driving means and the separating means of the clamp.

### SUMMARY

In order to solve the above-mentioned technical problems, the present disclosure provides a system for clamping a tissue, which may include an improved mechanism to capture a valve leaflet more stably and reliably.

The invention is a heart valve clamp device as defined by the features of the independent claim.

Specifically, the present disclosure employs the following solutions:

A system for clamping a tissue includes:
a fixing device including a clamp mechanism for closing a tissue, and a support mechanism carrying the clamp mechanism, the support mechanism including a drive assembly for driving the clamp mechanism to open and close;
a delivery control device including a push shaft for introducing the fixing device to a target location and a clutch mechanism for enabling the push shaft and the fixing device to be separably connected, and
the clamp mechanism includes a pair of closure members, a guide slot including at least a nonlinear segment in each of the pair of closure members, the support mechanism is provided with slot driving members, each of which is at least partially located in one of the guide slots and slidable in the one of the guide slots, so as to drive two closure clamping portions to relatively move towards or away from each other.

Further, the clamp mechanism may include the pair of closure members and a pair of capture members, each capture member in one-to-one correspondence with a respective one of the closure members; and the pair of closure members may include closure connecting portions and the closure clamping portions arranged to cooperate with the pair of capture members to clamp the tissue; and
the guide slots may be formed in the closure connecting portions.

Further, the support mechanism may include a fixed connecting assembly, and the slot driving members at least partially located in the guide slots may be provided on the fixed connecting assembly, and the drive assembly may be connected to the two closure clamping portions, and may be arranged to allow each of the slot driving members to slide in the respective one of the guide slots when the drive assembly moves relative to the fixed connecting assembly.

Further, each of the guide slots may include at least two slot regions in communication with each other, and a radian of the first slot region of the two slot regions may be greater than a radian of the second slot region of the two slot regions.

Further, each of the guide slots may further include a third slot region in communication with the second slot region and having a radian greater than the radian of the second slot region, and the second slot region may be located between the first slot region and the third slot region, respectively.

Further, the clutch mechanism may include an actuator rod connected to the drive assembly in a non-rotatable and axially separable manner such that rotation of the actuator rod directs the drive assembly to move axially; and
the actuator rod may be configured to disengage the clutch mechanism from the fixing device when the actuator rod is moved proximally.

Further, the support mechanism may include a fixed connecting assembly and the drive assembly which may be moveable relative to the fixed connecting assembly, and the fixed connecting assembly may include a base clutching end;
the clutch mechanism may include a coupling seat separably connected to the base clutching end, and an engagement member releasably connecting the base clutching end and the coupling seat, and the actuator rod may provide a driving force for the drive assembly; and
the actuator rod may include an actuator rod clutching end connected to the drive assembly in an axially separable and non-rotatable manner, the actuator rod may be moveable relative to the engagement member between a first position and a second position, and when the actuator rod moves proximally from the second position, an actuator rod supporting portion can drive the engagement member to move proximally such that the coupling seat can be separated from the base clutching end.

Further, the coupling seat may include a coupling seat connecting end, a coupling seat clutching end, and a coupling seat inner cavity defined through the coupling seat, the engagement member may be provided in the coupling seat inner cavity, the coupling seat clutching end may be connected to the base clutching end via the engagement member, and the coupling seat connecting end may be connected to a delivery device.

Further, the delivery control device may further include a manipulation wire for controlling capture members of the clamp mechanism to open and close, the engagement member may include a manipulation wire limiting groove for preventing separation of the manipulation wire when the coupling seat is connected to the base clutching end.

Further, the fixed connecting assembly may include a base housing with a base inner cavity formed therein, a base threaded portion may be provided in the base inner cavity, the drive assembly may include a drive shaft, the drive shaft may include a drive shaft threaded portion cooperating with the base threaded portion, and wherein a lead angle at which the base threaded portion is cooperating with the drive shaft threaded portion may be less than a friction angle.

The present disclosure achieves the following beneficial effects.
1) By improving driving methods and employing the guide slot(s) having nonlinear segment(s) for cooperative driving, and changing an opening angle and a capturing distance of the closure member, the present disclosure makes it easy to capture a valve leaflet, achieves more contact with the valve leaflet, and makes the valve leaflet to be captured more firmly.
2) By sequentially providing the first guide slot region, the second guide slot region and the third guide slot region, the closure member may have a relatively smaller torque and a faster change process during opening at an initial position and at a position with a maximum opening angle compared with the intermediate capturing process, and meanwhile may have a relative slow angular change at the capturing process, thereby facilitating fine operation by the operator, and improving system reliability.
3) While an actuator rod clutching end is separated from a transmission rod clutching end, the coupling seat can also be synchronously separated from the base clutching end. The whole separation action can be accomplished by one step, and there is no need to separate the components with several complicated steps.
4) When the base housing or the drive shaft is stopped, no axial displacement will occur if the axial force is only applied to the base housing or the drive shaft. Therefore, the present disclosure realizes self-locking without using a spring piece or other structures, such as those used in the prior art, and can hold its position by thread fit.

### BRIEF DESCRIPTION OF THE DRAWINGS

In order to illustrate the technical solutions in the embodiments of the present disclosure more clearly, the drawings for the description of the embodiments or the prior art are briefly described below. Apparently, the accompanying drawings described below are merely some embodiments of the present disclosure, and those of ordinary skill in the art may still derive other drawings from these accompanying drawings without creative efforts.
FIG. 1 shows a schematic structural view, in which a clamp mechanism according to one example of the present disclosure is located at a mitral valve;

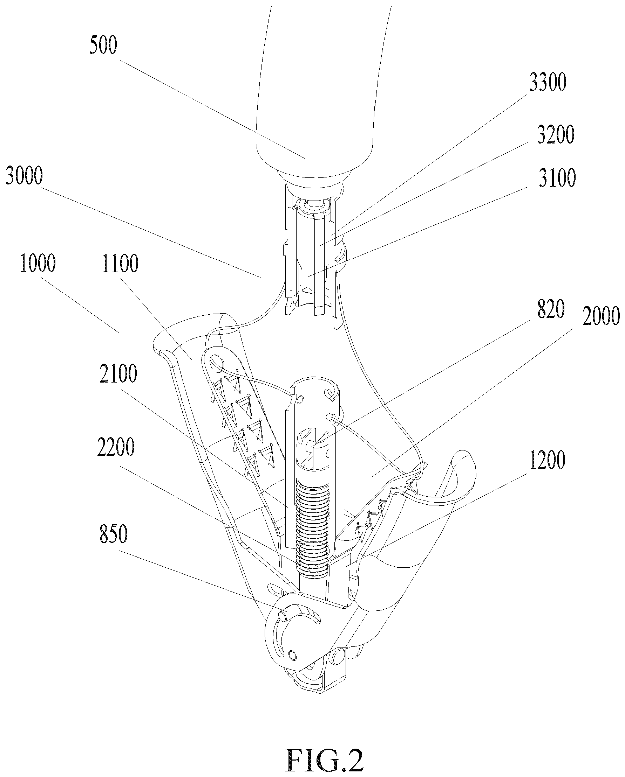

FIG. 2 is a schematic view of an overall structure according to the present disclosure;
FIG. 3 is a partial sectional view of an overall structure according to the present disclosure;
FIG. 4 is a principle schematic view illustrating a capture member in different states, according to the present disclosure;
FIG. 5(a) is a side view of a closure member according to the present disclosure;
FIG. 5(b) is a schematic structural view of a closure member according to the present disclosure;
FIGS. 6(a)-(d) each are schematic views illustrating the principle of a closure member according to the present disclosure;
FIG. 7(a) is a schematic structural view of a capture member according to the present disclosure;
FIG. 7(b) is a sectional view of a capture member according to an implementation of the present disclosure;
FIG. 7(c) is a sectional view of a capture member according to another implementation of the present disclosure;
FIGS. 8(a)-(e) each illustrate a part of a support mechanism according to the present disclosure;
FIGS. 9(a)-(c) each illustrate a movement of a closure member and capture member in a clamping process according to the present disclosure;
FIGS. 10(a)-(d) each illustrate a part of a clutch mechanism according to the present disclosure;
FIG. 11 is a partial enlarged view illustrating a disengagement position of a manipulation wire;
FIGS. 12(a)-(d) each illustrate a movement process when a clamp mechanism releases a closure member according to the present disclosure; and
FIGS. 13(a)-(d) each illustrate a process of capture and fixation movement of a clamp mechanism according to the present disclosure.

### DETAILED DESCRIPTION

The technical solutions of the embodiments of the present disclosure are clearly and completely described below with reference to the accompanying drawings. Apparently, the described embodiments are merely a part rather than all of the embodiments of the present disclosure. In the description of the embodiment, the term "proximal end" refers to an end proximal to the operator, while the term "distal end" refers to an end distal to the operator. All other embodiments obtained by those of ordinary skill in the art based on the embodiments of the present disclosure without making inventive efforts shall fall within the scope of protection of the present disclosure.

### Embodiment 1

In an embodiment, a system for clamping a tissue is provided, specifically including:
a fixing device including a clamp mechanism 1000 for closing a tissue, and a support mechanism 2000 for mounting the clamp mechanism 1000, the support mechanism 2000 including a drive assembly 2200 for driving the clamp mechanism 1000 to open and close; and
a delivery control device including a push shaft 600 for pushing the fixing device to a target location and a clutch mechanism 3000 for enabling the push shaft 600 and the fixing device to be separably connected.

The clamp mechanism 1000 includes a pair of closure members 1100. A guide slot 1121 including at least a nonlinear segment is formed in each of the pair of closure members 1100. The support mechanism 2000 is provided with slot driving members 850, each of which is at least partially located in one of the guide slots 1121. Each of the slot driving members 850 is relatively slidable in the one of guide slots 1121, so as to drive two closure clamping portions 1110 to move towards or away from each other.

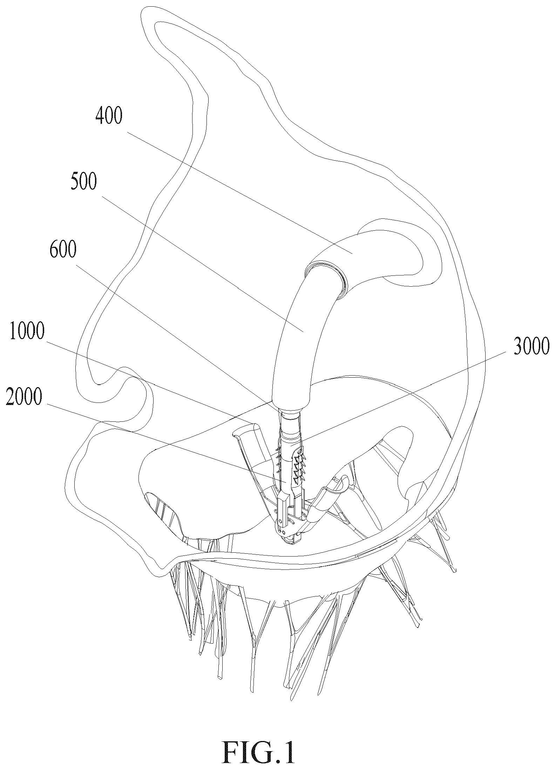

Specifically, the clamp mechanism 1000 in the example includes a pair of closure members 1100 and a pair of capture members 1200, each capture member 1200 in one-to-one correspondence with one of the closure members 1100. The closure members 1100 can be opened and closed by the drive assembly 2200. The capture members 1200 can be opened and closed by a manipulation wire 610. In order to clamp the tissue, the closure members 1100 maybe extended inward, while the capture members 1200 may be extended outward, to achieve cooperation with each other. The system may be used for clamping of a heart valve leaflet as an implementation to describe a specific working principle of the fixing device for clamping the tissue. Referring to FIG. 1, the fixing device in the example is delivered to a target location in a heart by the delivery control device. Specifically, the delivery control device includes a push shaft 600 for introducing the fixing device to the target location and a clutch mechanism 3000 for allowing the push shaft 600 and the fixing device to be separably connected. In one example of the present disclosure, the push shaft 600 may be a rod-like body with an inner cavity or a hollow tubular body, which is made of a biocompatible material. In the example, an engagement shaft 820 is shaped as a circular rod or a circular tube. The push shaft 600 has a smooth surface to avoid damage to a valve leaflet or a chordae tendineae. The push shaft 600 first enters a surgical channel together with a catheter 500. After reaching a vicinity of the lesion, the push shaft 600 may be advanced out of the catheter 500 to deliver the fixing device to the mitral valve. Preferably, a distal end of the fixing device, namely a distal end of the clamp mechanism 1000, may be coated with a protective covering layer. The protective covering layer is made of a biocompatible material and completely coats a periphery of the clamp mechanism 1000. The protective covering layer can prevent a damage of the device to the tissue. When the fixing device stays in the heart as an implant, an outer surface of the fixing device can be completely protected by the protective covering layer.

After the fixing device arrives at the valve, regions of the anterior valve leaflet and the posterior valve leaflet of the heart valve (which cannot be properly closed) are clamped by cooperation of the closure members 1100 and the capture members 1200 of the clamp mechanism 1000 in the embodiment. As a result, the leaflets that cannot be closed properly are clamped together, such that the mitral valve can be tightly closed or closed with a smaller opening, thereby treating or relieving "mitral regurgitation".

After the mitral valve is clamped, the fixing device is separated from the delivery control assembly by the clutch mechanism 3000. The fixing device remains at the lesion to fix the valve.

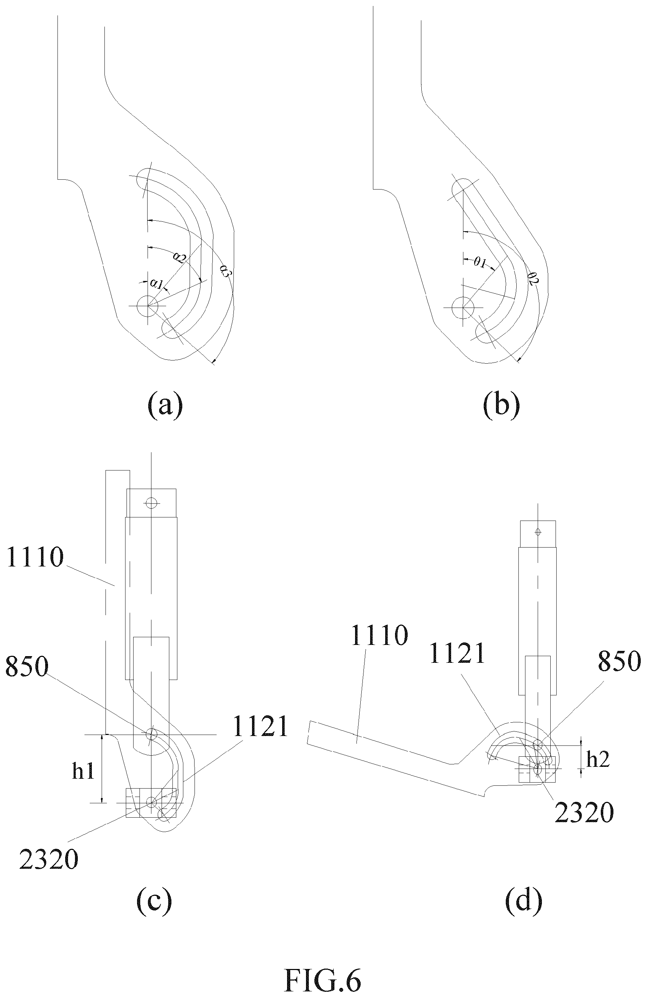

The guide slot 1121 includes at least two guide slot regions (namely a first slot region and a second slot region) in communication with each other. A radian of the first slot region may be greater than a radian of the second slot region. FIG. 6(a) illustrates an implementation of the guide slot according to a first example of the present disclosure. Herein, the guide slot may be divided into a first slot region, a second slot region, and a third slot region extending from a distal end to a proximal end. In the example, the first slot region and the third slot region are arc segments, while the second slot region is a linear segment. With the arc feature, the turning angle can be changed in a larger range. Meanwhile, it has a small torque with a nonlinear variation in a small range, and a dead point where the torque is zero. With a linear feature, the torque is large, and changes linearly and stably. It can be changed in a larger range without a dead point, but the turning angle can be changed in a smaller range. Therefore, with the guide slot 1121 defined as a first curved slot region, a second linear slot region, and a third curved slot region, the closure member 1110 may experience a relatively smaller torque and a faster change process during opening at an initial position and at a position with a maximum opening angle, compared with the intermediate capturing process, and meanwhile has a relative slow angular change at the capturing process, , thereby facilitating fine operation by the operator, and improving system reliability.

Referring to FIG. 6(a) and FIGS.6(c)-(d), the slot driving member 850 is shaped as a circular push shaft, and located in the guide slot 1121. A width of the guide slot 1121 is matched with an outer diameter of the slot driving member 850. At an initially unopened position, the slot driving member 850 is located at the most proximal end, as shown in FIG. 6(c), namely the most proximal end of the third slot region. A distance between the slot driving member 850 and a closure member matching portion 2320 is h1. During opening of the closure clamping portion 1110, the slot driving member 850 slides in the third slot region toward the distal end, and enters the second slot region from a distal-most end of the third slot region. In such a case, the closure clamping portion 1110 is rotated by an angle of α1. When the slot driving member 850 further slides to a distal-most end of the second slot region, namely at an extreme end of the linear segment, the closure clamping portion 1110 is rotated by an angle of α2. A distance between the slot driving member 850 and the closure member matching portion 2320 is h2, as shown in FIG. 6(d). When the slot driving member 850 further slides toward a distal end in the first slot region and arrives at a distal-most end of the guide slot 1121, the closure clamping portion 1110 is rotated by an angle of α3. Herein, the α1, the α2, the α3, the h1, and the h2 can be selected and ranged according to an actual need. In an embodiment, the angle α1 is preferably in a range of 30° to 45°, more preferably in a range of 35° to 40°, and even more preferably 40°. The angle α2 is preferably in a range of 55° to 70°, more preferably in a range of 60° to 70°, and even more preferably 65°. The angle α3 is preferably in a range of 120° to 150°, more preferably in a range of 125°to 135°, and even more preferably 130°. The distance h1 is preferably in a range of 15 mm to 16 mm, and more preferably 15.5 mm. The distance h2 is preferably in a range of 5 mm to 7 mm, and more preferably 6 mm. As can be seen from the above descriptions, to rotate the closure clamping portion 1110 by the angle of α2, the actuator needs to drive the closure member matching portion 2320 to move relative to the slot driving member 850 by a distance of h2-h1. When the angle α2 is 65°, h1 is 15.5 mm, and h2 is 6 mm, namely the two closure clamping portions 1110 are opened relatively at 130°, the closure member matching portion 2320 moves by a distance of 9.5 mm. With the cooperation of the first curved slot region, the second linear slot region and the third curved slot region, the closure member has the faster change process during opening at the initial position and at the position with the maximum opening angle compared with the intermediate capturing process, and meanwhile has a relative slow angular change at the capturing process, such that the capture is more stable. Compared with a common linear slot, the clamp mechanism 1000 may achieve a shorter driving distance to reach a preset capturing angle. Moreover, at a position to perform fine operation, the clamp mechanism of the present closure may be safer and more reliable than that with a curved slot.

FIG. 6(b) illustrates an implementation of the guide slot 1121 according to another embodiment of the present disclosure. The guide slot is divided into a first slot region and a second slot region from a distal end to a proximal end of the guide slot. As shown, the slot region is an arc segment, and the second slot region is a linear segment. This example is mainly intended to facilitate quick closure from the capturing position to the position with the maximum opening angle. In the tissue capturing process, the angular changing rate is reduced at the capturing segment by the linear segment, such that the capture is more stable. Specifically, from the most proximal end of the guide slot 1121 to an intersection between the first slot region and the second slot region, the slot driving member may traverse along a varying angle of θ1, namely the slot driving member 850 may traverse the varying angle of θ1 in the linear slot region of the guide slot 1121. With a further movement to the most distal end, the single closure clamping portion 1110 has an opening angle of θ2. Preferably, the angle θ1 is in a range of 40° to 60°, and the angle θ2 is in a range of 120° to 140°.

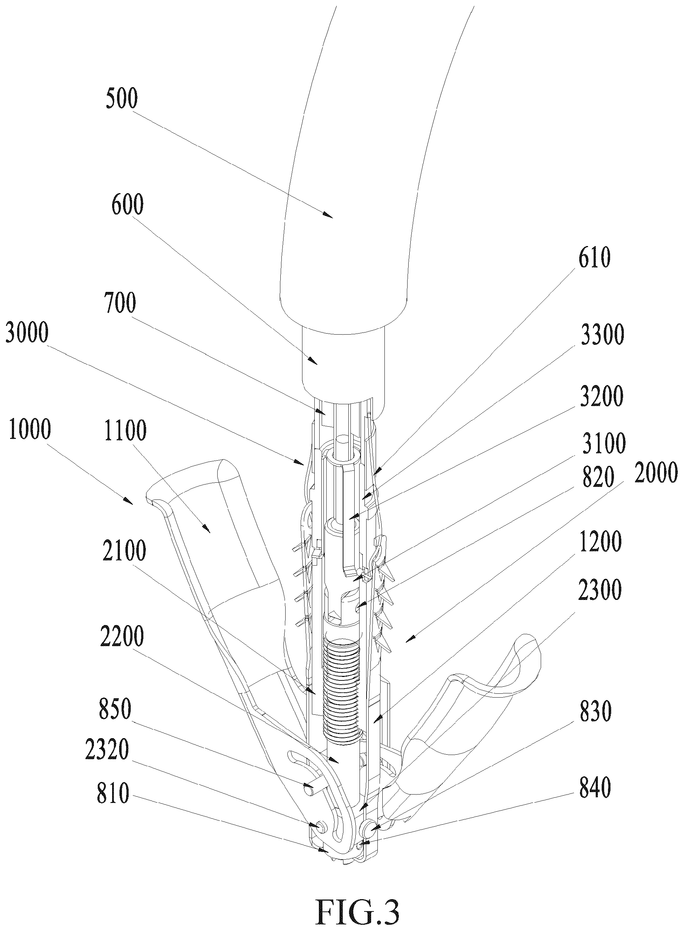

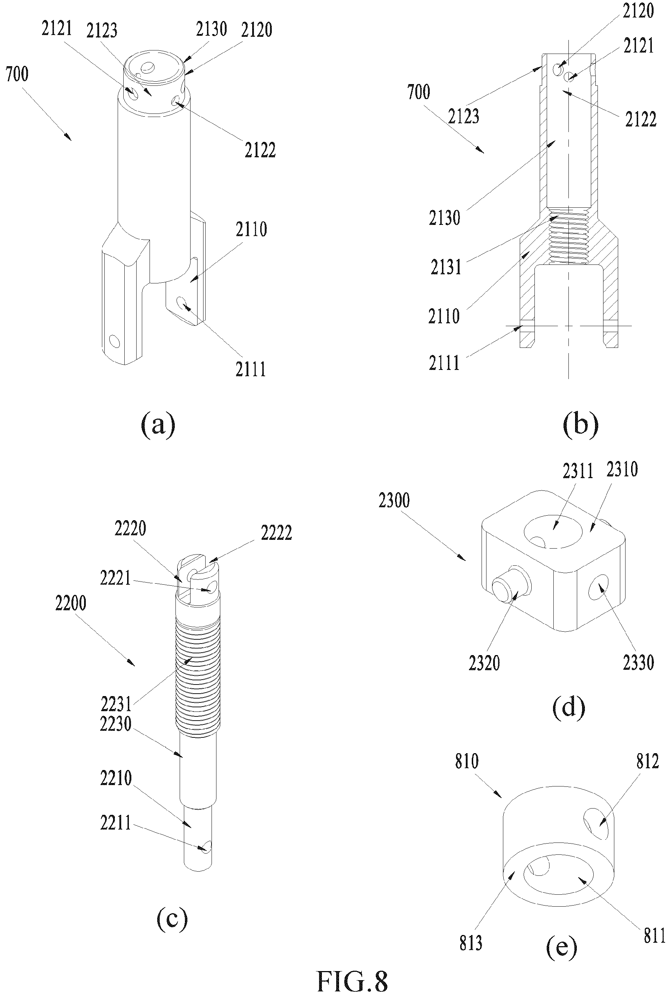

Referring to FIG. 3 and FIGS. 8(a)-(e), the drive assembly 2200 includes a movable seat 2300. The movable seat 2300 specifically includes a drive connecting member 2310. Side surfaces of the drive connecting member 2310 include first mounting surfaces and second mounting surfaces perpendicular to each other. The drive connecting member 2310 is hinged to the closure connecting portion 1120 through the closure member matching portion 2320 on the first mounting surfaces. This allows a movement of the closure member matching portion 2320, and further allows a relative movement between each guide slot 1121 and slot driving member 850.

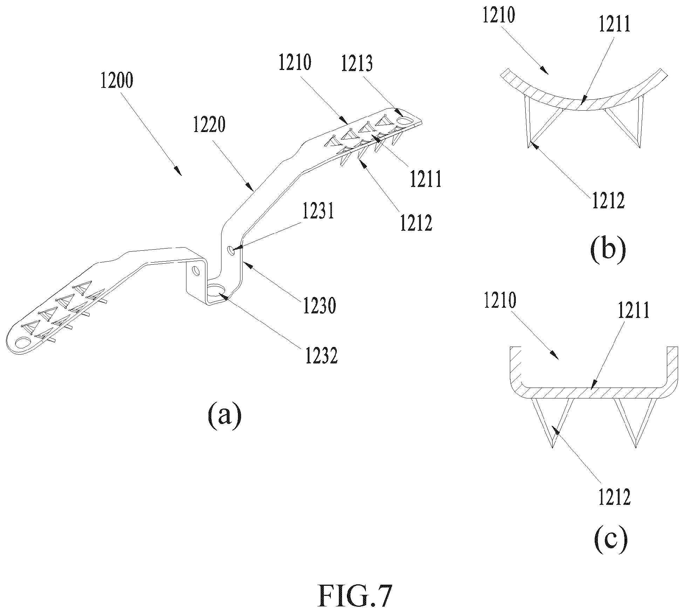

Further, referring to FIGS. 7, each one of the capture members 1200 of the disclosure includes a rigid capturing portion 1210, a flexible connecting portion 1220, and a capture connecting portion 1230 that are connected sequentially. The flexible connecting portion 1220 is located between the rigid capturing portion 1210 and the capture connecting portion 1230. The second mounting surfaces are fixedly connected to the capture connecting portion 1230.

In the example, the capture member can cooperate with the closure member to capture a moving valve leaflet. A captured valve leaflet may be located between the closure member and the capture member. The capture connecting portion may be connected to the second mounting surfaces. The capture member is presented as spreading wings of a bird in a natural state. The flexible connecting portion is specifically a deformable component with a certain resilience force. By applying an external turning force to the rigid capturing portion, the flexible connecting portion elastically deforms to change an angle between the rigid capturing portion and an axial direction, thereby achieving turning of the capture member. When a moving valve leaflet to be captured is located above the closure member at a same side with the capture member, the elastic flexible connecting portion may be instantaneously restored to an initial shape in a natural state once the external turning force is removed. In such a case, the valve leaflet can be captured between the closure member and the rigid capturing portion. In the valve capture process, the rigid capturing portion is mainly used to fix the moving valve leaflet. The structure of the rigid capturing portion needs to have a certain rigidity, so as to prevent the captured valve leaflet from escaping from the capture member.

More preferably, the rigid capturing portion 1210 includes a rigid surface 1211, and capturing barbules 1212 provided outside the rigid surface. The rigid surface 1211 has uniformly thick and is bent.

The rigid surface 1211 has a rigid section, which is curved as shown in FIG. 7(b) or bent as shown in FIG. 7(c). In the structure, the bent or curved portion is similar to a reinforcing rib formed on a main body. Consequently, the sheet-like thin-wall section has a high flexural coefficient and the rigidity of the rigid capturing portion is improved, without need of an additional component for reinforcing the rigidity. Moreover, in the example, when pushing and pulling the manipulation wire in the delivery control assembly, the manipulation wire applies a force to the capture member where the guide wire orifice 1213 is located, thereby achieving turning of the capture member.

In the example, the two capture members 1200 share one capture connecting portion 1230. The capture connecting portion 1230 is U-shaped, with symmetrical vertical portions being respectively and fixedly connected to the second symmetrical mounting surfaces of the drive connecting member 2310. The capture connecting portion 1230 may be fixedly connected to the drive connecting member 2310 by hinging, riveting or welding, such that the capture member 1200 may move axially with the drive connecting member 2310.

Take the slot driving members 850 as a reference point. When the drive connecting member 2310 axially moves close to the slot driving member 850, the closure member 1100 is opened and turned, and the overall capture member 1200 also axially moves close to the slot driving member 850. If an external turning force toward the slot driving member 850 is applied to the rigid capturing portion of the capture member 1200 at the same time, the capture member 1200 is turned toward the slot driving member 850. When a suitable valve leaflet contacts the opened closure member 1100, the external turning force applied on the capture member 1200 can be removed, and the valve leaflet can be clamped between the closure member 1100 and the capture member 1200.

In an embodiment, the capture member 1200 is expanded in the natural state. The manipulation wire 610 is arranged to constrict the capture member 1200 to an unexpanded state. By removing a constraint force of the manipulation wire 610, the capture member 1200 can be turned. In another embodiment, the capture member 1200 may also be a flexible member, and may be directly turned under pushing of the manipulation wire 610 for capture.

Further, when the drive connecting member 2310 starts to move axially away from the slot driving member 850, the closure member 1100 performs closing and turning movement together with the captured valve leaflet and the opened capture member 1200. Meanwhile, the drive connecting member 2310 also moves axially away from the slot driving member 850 together with the capture member 1200. In this case, the capture member 1200 and the valve leaflet have or trend to have a relative displacement. A pressure arising from an elastic deformation of the capture member 1200 is applied to the valve leaflet. As a consequence, a friction force occurs on a contact surface between the capture member 1200 and the valve leaflet that have the relative displacement. A direction that the capture member 1200 applies the friction force to the valve leaflet refers to a direction toward the drive connecting member 2310, such that the capture member "pulls" the valve leaflet. By increasing a friction force or barbs features, such as the capturing barbs 1212 in the embodiment, on the rigid capturing portion of the capture member 1200, the valve leaflet "pulling" effect will be more evident. Compared with the proximal element that is merely turned in the background art, the present disclosure may provide a connection between the valve leaflet and the fixing device more firm.

Further, referring to FIG. 3, the closure clamping portion 1110 in the embodiment includes a free end and a connecting end connected to the closure connecting portion 1120. In a direction from the connecting end to the free end, an outer surface of the closure clamping portion 1110 at least partially tends to shrink toward an inner side. Due to the closure clamping portion 1110 having a concave shape with a recess, a contact area with the valve leaflet can be increased when the valve leaflet is clamped. When the closure clamping portion clamps the valve leaflet in cooperation with the capture member 1200, the valve leaflet clamped in the recess of the closure clamping portion 1110 can limit a radial displacement of the fixing device on the valve leaflet. With a flange, a damage of an edge of the closure clamping portion 1110 to the valve leaflet can be prevented. After the valve leaflet is closed by the fixing device, due to the concave bend of the closure clamping portion 1110, end portions at two sides of the closure clamping portion are formed into a receiving portion, which makes the valve leaflet to be clamped more firmly in an axial direction. In addition, the clamp mechanism 1000 in other embodiments of the present disclosure can also be arranged to relieve or treat "tricuspid regurgitation". That is, in addition to the original pair of closure members 1100 and the original pair of capture members 1200, one more additional closure member and additional capture member are added to treat the "tricuspid regurgitation". The principle and structure for treating the tricuspid regurgitation are the same as those for treating the mitral regurgitation, and will not be repeated herein. It can be understood that other embodiments of the present disclosure can be apply to clamp several sheet-like tissues in other minimally invasive surgeries in other, and a number of the closure members 1100 and a number of the capture members 1200 are determined according to an actual use requirement.

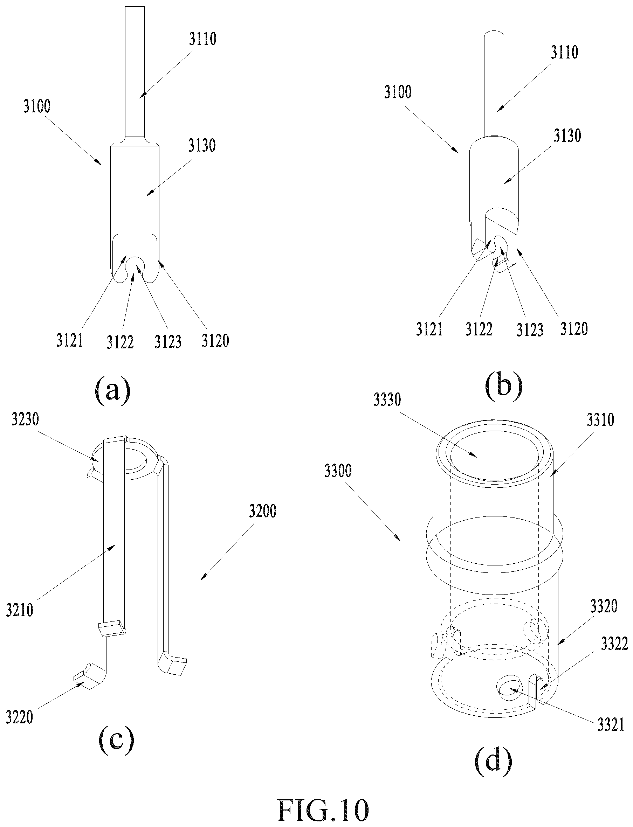

In the embodiment, the clutch mechanism 3000 includes an actuator rod 3100 connected to the drive assembly 2200 in a non-rotatable and axially separable manner. By rotating the actuator rod 3100, the drive assembly 2200 is driven to move axially. The actuator rod 3100 is arranged to be preset to drive the clutch mechanism 3000 to disengage from the fixing device when the actuator rod is moved proximally. Following descriptions are made to a separation principle between the clutch mechanism 3000 and the support mechanism 2000 in combination with FIG. 3, FIGS. 8 and FIGS. 10.

The support mechanism 2000 for carrying the clamp mechanism 1000 includes a fixed connecting assembly 2100 and a drive assembly 2200 moveable relative to the fixed connecting assembly 2100. Specifically, the drive assembly 2200 includes a push shaft 2230. The push shaft 2230 includes a push shaft threaded portion 2231 in cooperation with a base threaded portion 2131. The push shaft 2230 may be directed axially by rotary movement between a drive output push shaft 2210 and a base housing. The drive output push shaft 2210 is rotated by applying a rotating torque to the actuator rod 3100 in the clutch mechanism 3000. Specifically, the actuator rod includes an actuator rod clutching end 3120 connected to the drive assembly 2200 in an axially separable and non-rotatable manner, and an actuator rod supporting portion 3130. Specifically, the drive assembly 2200 includes a transmission rod clutching end 2220. The actuator rod clutching end 3120 is non-rotatably connected to the transmission rod clutching end 2220. When a tensile force between the actuator rod clutching end 3120 and the transmission rod clutching end 2220 is greater than a preset value, the actuator rod clutching end 3120 is disengaged from the transmission rod clutching end 2220. Since a lead angle of a spiral annular groove is less than a friction angle of a contact surface between two spiral grooves, when the base housing or the drive shaft 2230 is stopped, no axial displacement will occur if an axial force is only applied to the base housing or the drive shaft 2230. Therefore, in order to disengage the actuator rod clutching end 3120 from the transmission rod clutching end 2220, the actuator rod 3100 may simply be directed proximally relative to the transmission rod grasping end 2220, thereby implementing disengagement from the drive assembly 2200 when the preset value is reached.

Specifically, an implementation for the disengagement of the fixing device from the drive assembly 2200 when the preset value is reached is as follows.

One of the actuator rod clutching end 3120 and the transmission rod clutching end 2220 is provided with a deformable buckle 3121, while the other one of the actuator rod clutching end and the transmission rod clutching end is provided with a connecting groove 2222. The deformable buckle 3121 may be made of an elastic biocompatible material, such as a biocompatible high polymer material. The engagement shaft 820 which can be cooperatively connected to the deformable buckle 3121 is provided in the connecting groove 2222. The engagement shaft 820 is inserted into an engagement shaft hole 222. Specifically, the deformable buckle 3121 is provided with a bayonet 3122 and a clamping hole 3123 matched with the engagement shaft 820 through the bayonet 3122. The actuator rod clutching end 3120 and the transmission rod clutching end 2220 are connected to the engagement shaft 820 through the deformable buckle 3121. When the tensile force between the actuator rod clutching end 3120 and the transmission rod clutching end 2220 is greater than a preset value, the deformable buckle 3121 is disengaged from the engagement shaft 820. In the embodiment, the deformable buckle 3121 is provided at the actuator rod clutching end 3120, while the connecting groove 2222 is provided at the transmission rod clutching end 2220. However, an opposite arrangement is also conceivable.

In order to ensure an outer housing of the support mechanism 2000 is separated from an outer housing of the clutch mechanism 3000 when the actuator rod clutching end 3120 is separated from the transmission rod clutching end 2220, specifically, the clutch mechanism 3000 in the embodiment is further provided with a coupling seat 3300 separably connected to the base clutching end 2120, and an engagement member 3200 for connecting the base clutching end 2120 and the coupling seat 3300. The actuator rod 3100 is arranged to be moveable relative to the engagement member 3200 between a first position and a second position. When the actuator rod 3100 moves toward the proximal end from the second position, an actuator rod supporting portion 3130 can drive the engagement member 3200 to move toward the proximal end as well, such that the coupling seat 3300 can be relatively separated from the base clutching end 2120. Therefore, when the actuator rod clutching end 3120 is separated from the transmission rod clutching end 2220, the coupling seat 3300 can also be synchronously separated from the base clutching end 2120.

Specifically, the coupling seat 3300 in the embodiment includes a coupling seat connecting end 3310, a coupling seat clutching end 3320, and a coupling seat inner cavity 3330 defined through the coupling seat 3300. The engagement member 3200 is provided in the coupling seat inner cavity 3330. The coupling seat clutching end 3320 is connected to the base clutching end 2120 via the engagement member 3200. The coupling seat connecting end 3310 is connected to a delivery device.

A main body of the base clutching end 2120 is an extension of a tubular base structure. The coupling seat clutching end 3320 can be sleeved on the base clutching end 2120, or inserted into the base clutching end 2120. Contact surfaces of the two ends are respectively referred to as a base matching surface and a coupling seat matching surface. A base clamping hole 2121 is formed on the base matching surface in a radial direction. Correspondingly, a coupling seat clamping hole 3321 having a same orientation is formed on the coupling seat matching surface in a radial direction. The coupling seat clutching end 3320 is provided with the coupling seat clamping hole 3321. The base clutching end 2120 is provided with the base clamping hole 2121 corresponding to the coupling seat clamping hole 3321. The engagement member 3200 includes buckles 3220, each of which pass through both the coupling seat clamping hole 3321 and the base clamping hole 2121, such that the coupling seat 3300 and the base clutching end 2120 are fixed relatively to one another. The buckle 3220 is arranged in such a manner that, when the engagement member 3200 moves toward the proximal end relative to the coupling seat 3300, the buckle 3220 gets away from the coupling seat clamping hole 3321 and/or the base clamping hole 2121, such that the coupling seat 3300 and the base clutching end 2120 can be separated from each other.

The buckle 3220 is made of a flexible material. When the engagement member 3200 moves toward the proximal end relative to the coupling seat 3300, the buckle 3220 deforms such that it can directly exit the coupling seat clamping hole 3321 and the base clamping hole 2121, without resilience to cause failure of the separation. The buckle 3220 is made of a biocompatible plastic or metal material that is not resilient after being bent.

Further, the engagement member 3200 in the embodiment further includes one claw bottom ring 3230 and claw connecting rods 3210. The number of the claw connecting rods 3210 is the same as the buckles 3220, and the claw connecting rods 3210 connect the buckles 3220 to the claw bottom ring 3230, respectively. The claw bottom ring 3230 is provided with a bottom ring opening. There may be three to six claw connecting rods 3210 that are uniformly connected to a side of the claw bottom ring 3230. Preferably, there are three claw connecting rods 3210. A movement of the buckle 3220 to a center is obstructed by a radial surface of the push shaft-like or rod-like joystick supporting portion 3130. It can prevent the buckle 3220 from accidentally separating from the base clamping hole 2121 and the coupling seat clamping hole 3321, and ensure the connection between the base clutching end 2120 and the coupling seat 3300.

The actuator rod 3100 further includes an actuator rod connecting end 3110 connected to a proximal end of the actuator rod supporting portion 3130. A proximal end of the actuator rod connecting end 3110 is connected to a drive source through the bottom ring opening. An outer diameter of the proximal end of the actuator rod supporting portion 3130 is greater than an inner diameter of the bottom ring opening.

The actuator rod supporting portion 3130 is shorter than each of the claw connecting rods 3210. An outer diameter of the actuator rod supporting portion 3130 is provided in such a manner that, when the actuator rod supporting portion 3130 is located in the coupling seat clamping hole 3321, an outer surface of the actuator rod supporting portion 3130 prevents the buckle 3220 from getting away from the coupling seat clamping hole 3321 and the base clamping hole 2121. Hence, the buckle 3220 deforms only when the actuator rod supporting portion 3130 contacts the claw bottom ring 3230 and further moves toward the proximal end.

On the basis of the above structure, to separate the base clutching end 2120 from the coupling seat 3300, the actuator rod 3100 needs to move toward the claw bottom ring 3230 under an axial force. When the claw bottom ring 3230 is squeezed by the actuator rod, the actuator rod supporting portion 3130 is disengaged from the buckle 3220 and no longer limits a radial movement of the buckle 3220. Under a condition of a sufficient external force, the buckle 3220 can be pulled out from the base clamping hole 2121 and the coupling seat clamping hole 3321, to achieve separation of the base clutching end 2120 from the coupling seat 3300.

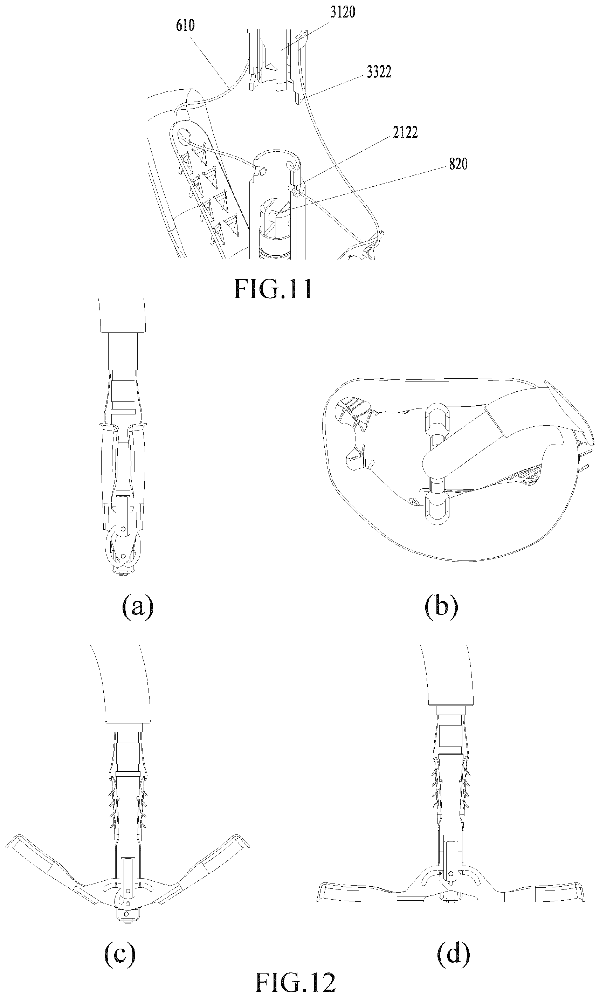

The above example is further described below. The coupling seat clutching end 3320 is sleeved on the base clutching end 2120. As shown in FIG. 11, the coupling seat 3300 includes a manipulation wire limiting groove 3322. The manipulation wire 610 for controlling the capture members 1200 of the clamp mechanism 1000 to open and close in the delivery control assembly includes an expanded head end. The expanded head end is provided in a base clutching end hole 2122. The minimal size of the expanded head end is greater than the manipulation wire limiting groove 3322 and less than the base clutching end hole 2122. When the coupling seat 3300 is connected the base clutching end 2120, the separation of the manipulation wire 610 is limited through the manipulation wire limiting groove 3322.

In an embodiment, the actuator rod 3100 is provided therein with a channel extending through the proximal end to a channel of the actuator rod clutching end 3120. The actuator rod 3100 in this embodiment is internally hollow and is different from those solid ones for threaded connection in the prior art. Due to the cooperation between the connecting groove 2222 and the deformable buckle 3121, and additionally a flexible push shaft for controlling a direction, an external operation structure for controlling turning can be omitted.

The structure and principle of the support mechanism 2000 are described below in combination with FIG. 3 and FIGS. 8. The support mechanism 2000 includes a fixed connecting assembly 2100 and a drive assembly 2200 moveable relative to the fixed connecting assembly 2100. A distal end of the drive assembly 2200 is connected to two closure members 1100, so as to control the closure members 1100 to open or close when the drive assembly 2200 moves relative to the fixed connecting assembly 2100. Specifically, the closure members 1100 of the clamp mechanism 1000 each include a closure connecting portion 1120 and include a closure clamping portion 1110. The closure clamping portions 1110 serve to cooperate with the capture members 1200 to clamp a tissue. The guide slots 1121 are formed in the closure connecting portion 1120. A slot driving member 850 at least partially located in the nonlinear guide slot 1121 is provided on the fixed connecting assembly 2100. The guide slot 1121 includes at least a nonlinear segment. The drive assembly 2200 is connected to the two closure clamping portions 1120, and is arranged to allow the slot driving member 850 to slide within the guide slot 1121 when the drive assembly 2200 moves relative to the fixed connecting assembly 2100, thereby driving the two closure clamping portions 1110 to relatively move close to or away from each other.

Further, the base housing is further provided with a base lug 2110 at the distal end. The slot driving member 850 is disposed outside the base lug 2110. By controlling the drive assembly 2200 to move relative to the slot driving member 850, the closure member 1100 is opened or closed.

Specifically, the fixed connecting assembly 2100 includes a base housing. A base inner cavity 2130 is formed in the base housing. A base threaded portion 2131 is provided in the base inner cavity 2130. The drive assembly 2200 includes a push shaft 2230. The push shaft 2230 includes a push shaft threaded portion 2231 in cooperation with the base threaded portion 2131. A lead angle at which the base threaded portion 2131 is cooperated with the push shaft threaded portion 2231 is less than a friction angle.

In the example, the push shaft threaded portion 2231 and the base threaded portion 2131 slidably cooperate with each other. They have a same thread pitch and a same sectional shape. By rotating either the base housing or the push shaft 2230, the base housing and the push shaft 2230 can move axially relative to each other. Since a lead angle of a spiral annular groove is less than a friction angle of a contact surface between the two spiral grooves, no axial displacement would occur when the base housing or the push shaft 2230 is stopped, if only an axial force is applied to the base housing or the push shaft 2230. Therefore, a self-locking function can be achieved without using a spring piece or other structures, unlike in the prior art, and can hold its position through a thread fit.

The drive assembly 2200 further includes a drive output push shaft 2210 disposed at a distal end of the drive shaft 2230 and having an outer diameter less than that of the drive shaft 2230. The drive connecting member 2310 is provided with a connection guide hole 2311 having an inner diameter matched with the drive output push shaft 2210. The drive output push shaft 2210 is axially and rotatably cooperated with the connection guide hole 2311. The drive assembly 2200 further includes a locating sleeve 810 disposed at a distal end of the drive connecting member 2310. The drive output push shaft 2210 extends to a locating sleeve mounting hole 811 of the locating sleeve 810 through the connection guide hole 2311, and fixedly connected to the locating sleeve 810.

As can be seen from the above structural descriptions, how the drive output push shaft 2210 is movably cooperated with the drive connecting member 2310 is disclosed in the embodiment. An output end of the drive output push shaft 2210 outputs thrust force and tensile force to the drive connecting member 2310, achieve axial movement of the drive connecting member 2310. However, as the drive output push shaft 2210 and the base housing are rotatably moved relative to each other while the drive connecting member 2310 and the base housing are not, the above structure can realize a rotary driving process without causing any rotation movement of the clamp mechanism to rotate, and the clamp mechanism can move stably in the axial direction. The locating sleeve 810 may be a tubular part. End surfaces of the locating sleeve 810 are axial end surfaces at two sides. The locating sleeve 810 and the output end of the drive output push shaft 2210 may be fixedly connected by either welding or interference fit, or mechanical connection. For example, the mechanical connection may be as follows. A locating sleeve positioning hole 812 may be formed on the locating sleeve 810 in a radial direction. A drive output push shaft positioning hole 2211 is formed, in a radial direction, on the output end of the drive output push shaft 2210 at the position where the output end of the drive output push shaft 2210 is cooperated with the locating sleeve 810. Pins 840 are respectively provided in each of the locating sleeve positioning hole 812 and the drive output push shaft positioning hole 2211.

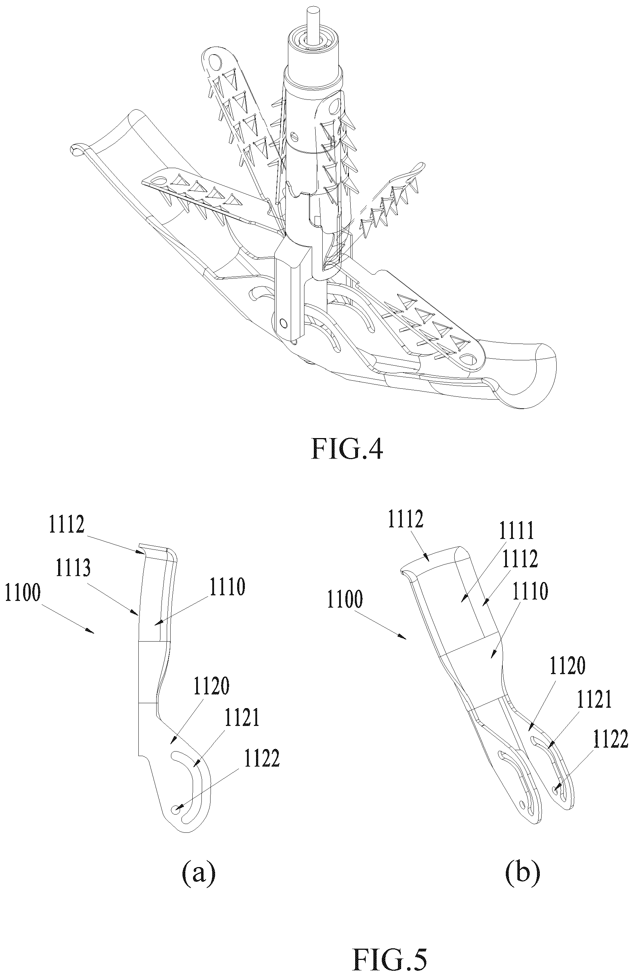

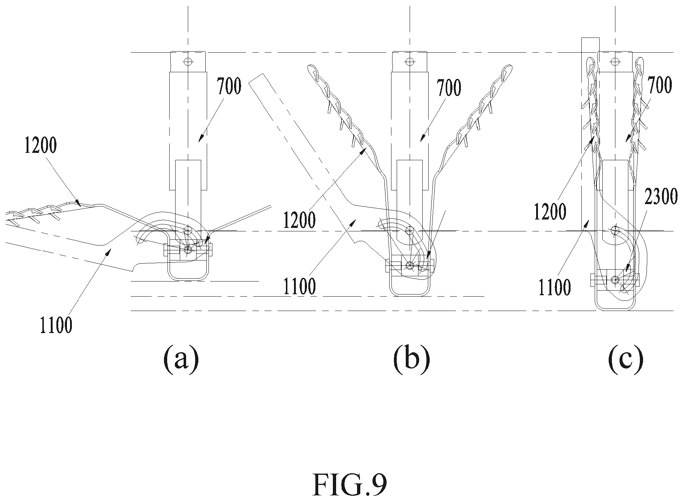

The clamp mechanism 1000 is described in the embodiment further referring to FIG. 4-9 and FIGS.12-13. Specifically, a distal end of the closure connecting portion 1120 is rotatably connected to the drive assembly 2200, in particular to a distal end of the drive assembly 2200. In the embodiment, the closure connecting portion 1120 is provided with a connecting portion push shaft hole 1122, and is connected to the drive assembly 2200 through a rotating push shaft. In other embodiments, other hinged methods may be conceivable. Before the fixing device is delivered to the target location, the closure clamping portion 1110 is in an initial state in which the slot driving member 850 is located at the proximal end of the guide slot 1121, and the closure clamping portion 1110 is closed, as shown in FIG. 12(a). Under driving of the drive assembly 2200, when the rotating joint between the drive assembly 2200 and the closure connecting portion 1120 moves toward the proximal end, i.e., the connecting portion push shaft hole 1122 moves toward the proximal end, the guide slot 1121 moves toward the proximal end synchronously due to the cooperation between the slot driving member 850 and the guide slot 1121. In such a case, the slot driving member 850, which is not moved, actually moves toward the distal end relative to the guide slot 1121. In order to make the closure clamping portion 1110 adapt to the change of distance, the slot driving member 850 rotates the guide slot 1121 in some extent relative to the connecting portion push shaft hole 1122, such that the closure clamping portion 1110 is rotated with the connecting portion push shaft hole 1122 as a center. In such a case, the closure clamping portion 1110 is turned outward with the connecting portion push shaft hole 1122 as an axial center, to enter the states as shown in FIGS. 12(b)-(c). By further driving the connecting portion push shaft hole 1122 to move toward the proximal end, the closure clamping portion 1110 can enter a state, in which the two closure clamping portions 1110 form an angle of 180° relative to each other, and the distance between the end portions achieves a maximum capturing distance, as shown in FIG. 12(d).

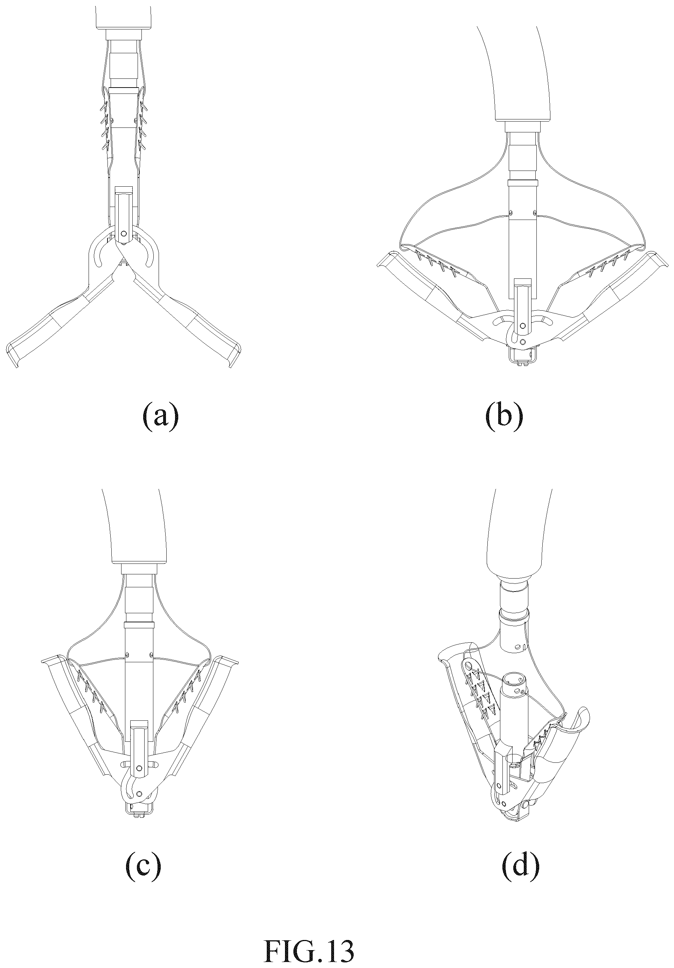

Due to the design of the guide slots 1121, the two closure clamping portions 1110 may be turned to form an angle of 180°, and further an obtuse angle as shown in FIG. 13(a). It can be applied when the fixing device needs to be withdrawn from the heart in case of inaccurate location or other problems. During the moving-out process, the two closure clamping portions 1110, which form an obtuse angle, tend to incline outward relative to the contact surfaces with the tissue and would not hook the tissue. Hence, the withdrawal can be smooth and safe. The specific principle of the process in which the closure clamping portion 1110 is cooperated with the guide slot 1121 through the slot driving member 850 to achieve the above opening angle will be described below in detail.

In the embodiment, throughout the opening and closing processes of the closure clamping portion 1110, the drive assembly 2200 moves relative to the slot driving member 850, while the slot driving member 850 does not move relative to the fixed connecting assembly 2100 connected therewith. Hence, during the process in which the closure clamping portion 1110 of the closure member 1100 is moved from an initial state to an open state, the closure connecting portion 1120 at the distal end moves toward the proximal end. Compared with the prior art where the clamping element is only opened and turned, the closure member 1100 in the present disclosure involves two kinds of movement in combination to allow the closure member 1100 to achieve a wider opening distance in the radial direction. Further, in the initial state of the closure clamping portion 1120, the closure connecting portion 1120 is not provided with other additional mechanisms. In such a case, a height of the overall fixing device can be lowered, and it is more convenient to turn during delivery. In view of this, the closure member 1100 can also be longer to facilitate a greater capturing distance.

After the closure clamping portion 1110 is cooperated with the capture member 1200 to clamp the tissue, the closure clamping portion 1110 further needs to be folded. When the closure clamping portion 1110 is folded, the closure connecting portion 1120 moves toward the distal end, and thus the closure clamping portion 1110 has an effect of being pulled toward the distal end. In such a case, a characteristic of "grabbing" movement may present in the folding process. When the tissue is clamped firmly, the valve leaflet on the closure clamping portion 1110 may be "pulled" in some extent, facilitating a more firm contact between the valve leaflet and the closure clamping portion 1110.

In order to illustrate a specific application of the device in the embodiment in surgery, an operation method of a system for clamping a tissue according to the disclosure is described in combination with specific structures in the embodiment, taking mitral valve repair as an example.

First step: pushing the fixing device connected to the push shaft 600, from a left atrium to a left ventricle through the mitral valve by the push shaft 600. In this case, the closure members 1100 of the clamp mechanism 1000 are in a closed state, as shown in FIG. 12(a).

Second step: adjusting relative positions of the valve fixing device and the mitral valve by the push shaft 600, such that the two closure members 1100 of the fixing device are respectively close to the anterior valve leaflet and the posterior valve leaflet of the mitral valve. Then, rotating the push shaft 2230. The base threaded portion 2131 is cooperated with the drive threaded portion 2231 to drive the drive connecting member 2310 to move toward the distal end. Taking the slot driving member 850 as a reference point, when the drive connecting member 2310 axially moves close to the slot driving member 850, the closure members 1100 are opened and turned to enter the states as shown in FIGS. 12(b)-(c). The closure members may also be further turned to enter the state as shown in FIG. 12(d). In this case, the distance between the end portions of the two closure members 1100 is maximized. After the two closure clamping portions 1110 form an angle of 180°, they may further be turned to form an obtuse angle as shown in FIG. 13(a), which can be applied when the fixing device needs to be withdrawn from the heart in case of inaccurate location or other problems. During the moving-out process, the two closure clamping portions 1110, which form an obtuse angle, tend to incline outward relative to the contact surfaces with the tissue and would not hook the tissue. Hence, the withdrawal can be smooth and safe.

Third step: After a valve leaflet is captured by the two closure members 1100, the capture member 1200 is turned toward the closure clamping portion 1110 by the manipulation wire 610, and thus the valve leaflet can be clamped between the closure member 1100 and the capture member 1200, as shown in FIG. 13(b).

Fourth step: When the drive connecting member 2310 starts to move axially away from the slot driving member 850, the closure member 1100 is closed and turned together with the captured valve leaflet and the opened capture member 1200. Meanwhile, the drive connecting member 2310 moves axially away from the slot driving member 850 together with the capture member 1200 to enter the states as shown in FIGS. 13(c)-(d). In this case, the capture member 1200 and the valve leaflet have a relative displacement or trend to have a relative displacement. A pressure arising from an elastic deformation of the capture member 1200 is applied to the valve leaflet. As a consequence, a friction force occurs on a contact surface between the capture member 1200 and the valve leaflet that have the relative displacement. The capture member 1200 applies the friction force to the valve leaflet in a direction towards the drive connecting member 2310, such that the capture member "pulls" the valve leaflet.

When the system for clamping a tissue according to the present disclosure is applied to repair the heart valve, the closure clamping portion has an effect of being pulled to the distal end, and a "grabbing" movement may occur in the folding process. When the tissue is clamped firmly, the valve leaflet on the closure clamping portion is "pulled". By employing the guide slot having the nonlinear segment for assisting driving, and increasing the opening angle and the capturing distance of the closure member, the fixing device makes it easy to capture the valve leaflet, increase the contact with the valve leaflet, and makes the valve leaflet to be captured more firmly.

Apparently, the terms such as "front", "rear", "upper", and "lower" as used in the description, refer to position and orientation relationships of parts and components in accordance with drawings for convenience of description for the purpose of simplicity and convenience. It should be understood that such orientation terms are not intended to limit the protection scope claimed by the present disclosure.

Any one or more of the embodiments of the present disclosure and any one or more of the features in the embodiments may be combined with each other without conflict.

## Claims

1. A heart valve clamp device, comprising:
a fixing device comprising a clamp mechanism (1000) for closing a heart valve, and a support mechanism (2000) carrying the clamp mechanism (1000), the support mechanism (2000) comprising a drive assembly (2200) for driving the clamp mechanism (1000) to open and close; and
a delivery control device comprising a push shaft (600) for introducing the fixing device to a target location and a clutch mechanism (3000) for enabling the push shaft (600) and the fixing device to be separably connected; wherein
the clamp mechanism (1000) comprises a pair of closure members (1100) each provided with a guide slot (1121) comprising at least a nonlinear segment, the support mechanism (2000) comprising slot driving members (850), each of which is at least partially located in the guide slots (1121) and is slidable in the guide slots (1121), so as to drive two closure clamping portions (1110) to move towards or away from each other.

2. The heart valve clamp device according to the previous claim, wherein the clamp mechanism (1000) comprises the pair of closure members (1100) and a pair of capture members (1200), each capture member in one-to-one correspondence with a respective one of the closure members (1100); the pair of closure members (1100) comprising closure connecting portions (1120) and the closure clamping portions (1110) in cooperation with the pair of capture members (1200) to clamp the heart valve; and the guide slot (1121) formed in the closure connecting portions (1120).

3. The heart valve clamp device according to the previous claim, wherein the support mechanism (2000) comprises a fixed connecting assembly (2100), the slot driving members (850) each of which is at least partially located in one of the guide slots (1121) are provided on the fixed connecting assembly (2100), and wherein the drive assembly (2200) is connected to the two closure clamping portions (1120) and is arranged to allow each of the slot driving members (850) to slide in the respective one of guide slots (1121) when the drive assembly (2200) moves relative to the fixed connecting assembly (2100).

4. The heart valve clamp device according to any one of the previous claims, wherein each of the guide slots (1121) comprises at least two slot regions in communication with each other, and a radian of a first slot region of the two slot regions is greater than a radian of a second slot region of the two slot regions.

5. The heart valve clamp device according to the previous claim, wherein each of the guide slots further comprises a third slot region in communication with the second slot region and having a radian greater than the radian of the second slot region, and wherein the second slot region is located between the first slot region and the third slot region.

6. The heart valve clamp device according to claim 1, wherein the clutch mechanism (3000) comprises an actuator rod (3100) connected to the drive assembly (2200) in a non-rotatable and axially separable manner such that rotation of the actuator rod (3100) directs the drive assembly (2200) to move axially; and the actuator rod (3100) is configured to disengage the clutch mechanism (3000) from the fixing device when the actuator rod (3100) is moved proximally.

7. The heart valve clamp device according to the previous claim, wherein the support mechanism (2000) comprises a fixed connecting assembly (2100) and the drive assembly (2200) is moveable relative to the fixed connecting assembly (2100), and wherein the fixed connecting assembly (2100) comprises a base clutching end (2120); the clutch mechanism (3000) comprises a coupling seat (3300) separably connected to the base clutching end (2120) and an engagement member (3200) connecting the base clutching end (2120) and the coupling seat (3300), and the actuator rod (3100) provides a driving force for the drive assembly (2200); and the actuator rod (3100) comprises a actuator rod clutching end (3120) connected to the drive assembly (2200) in an axially separable and non-rotatable manner, the actuator rod (3100) configured to be moveable relative to the engagement member (3200) between a first position and a second position, and when the actuator rod (3100) is moved proximally from the second position, an actuator rod supporting portion (3130) drives the engagement member (3200) to move proximally as well, such that the coupling seat (3300) is separated from the base clutching end (2120).

8. The heart valve clamp device according to the previous claim, wherein the coupling seat (3300) comprises a coupling seat connecting end (3310), a coupling seat clutching end (3320), and a coupling seat inner cavity (3330) defined through the coupling seat (3300), the engagement member (3200) is provided in the coupling seat inner cavity (3330), the coupling seat clutching end (3320) is connected to the base clutching end (2120) via the engagement member (3200), and the coupling seat connecting end (3310) is connected to a delivery device.

9. The heart valve clamp device according to the previous claim, wherein the delivery control device further comprises a manipulation wire (610) for controlling capture members (1200) of the clamp mechanism (1000) to open and close, and the coupling seat (3300) comprises a manipulation wire limiting groove (3322) for restricting separation of the manipulation wire (610) when the coupling seat (3300) is connected to the base clutching end (2120).

10. The heart valve clamp device according to claim 1, wherein the fixed connecting assembly (2100) comprises a base housing with a base inner cavity (2130) formed therein, a base threaded portion (2131) in the base inner cavity (2130), the drive assembly (2200) comprising a drive shaft (2230), the drive shaft (2230) comprising a threaded portion (2231) cooperating with the base threaded portion (2131), and wherein a lead angle at which the base threaded portion (2131) is cooperating with the drive portion (2231) is less than a friction angle.

## Patentansprüche

1. Herzklappenklemmvorrichtung, umfassend:
eine Befestigungsvorrichtung, die einen Klemmmechanismus (1000) zum Schließen einer Herzklappe und einen Stützmechanismus (2000), der den Klemmmechanismus (1000) trägt, umfasst, wobei der Stützmechanismus (2000) eine Antriebsbaugruppe (2200) zum Antreiben des Klemmmechanismus (1000) zum Öffnen und Schließen umfasst; und
eine Abgabesteuervorrichtung, die eine Druckwelle (600) zum Einführen der Befestigungsvorrichtung an eine Zielstelle und einen Kupplungsmechanismus (3000) umfasst, um zu ermöglichen, dass die Druckwelle (600) und die Befestigungsvorrichtung trennbar verbunden sind; wobei
der Klemmmechanismus (1000) ein Paar Verschlusselemente (1100) umfasst, die jeweils mit einem Führungsschlitz (1121) bereitgestellt sind, der zumindest ein nichtlineares Segment umfasst, wobei der Stützmechanismus (2000) Schlitzantriebselemente (850) umfasst, von denen sich jedes zumindest teilweise in den Führungsschlitzen (1121) befindet und in den Führungsschlitzen (1121) verschiebbar ist, um zwei Verschlussklemmabschnitte (1110) anzutreiben, um sich aufeinander zu oder voneinander weg zu bewegen.

2. Herzklappenklemmvorrichtung nach dem vorhergehenden Anspruch, wobei der Klemmmechanismus (1000) das Paar Verschlusselemente (1100) und ein Paar Einfangelemente (1200) umfasst, wobei jedes Einfangelement in Eins-zu-Eins-Entsprechung mit einem jeweiligen der Verschlusselemente (1100) ist; wobei das Paar Verschlusselemente (1100) Verschlussverbindungsabschnitte (1120) und die Verschlussklemmabschnitte (1110) in Kooperation mit dem Paar Einfangelemente (1200) umfasst, um die Herzklappe zu klemmen; und der Führungsschlitz (1121) in den Verschlussverbindungsabschnitten (1120) gebildet ist.

3. Herzklappenklemmvorrichtung nach dem vorhergehenden Anspruch, wobei der Stützmechanismus (2000) eine feste Verbindungsbaugruppe (2100) umfasst, wobei die Schlitzantriebselemente (850), von denen sich jedes zumindest teilweise in einem der Führungsschlitze (1121) befindet, an der festen Verbindungsbaugruppe (2100) bereitgestellt sind, und wobei die Antriebsbaugruppe (2200) mit den zwei Verschlussklemmabschnitten (1120) verbunden ist und angeordnet ist, um jedem der Schlitzantriebselemente (850) zu ermöglichen, in dem jeweiligen der Führungsschlitze (1121) zu gleiten, wenn sich die Antriebsbaugruppe (2200) relativ zu der festen Verbindungsbaugruppe (2100) bewegt.

4. Herzklappenklemmvorrichtung nach einem der vorhergehenden Ansprüche, wobei jeder der Führungsschlitze (1121) zumindest zwei Schlitzbereiche in Kommunikation miteinander umfasst und ein Bogenmaß eines ersten Schlitzbereichs der zwei Schlitzbereiche größer als ein Bogenmaß eines zweiten Schlitzbereichs der zwei Schlitzbereiche ist.

5. Herzklappenklemmvorrichtung nach dem vorhergehenden Anspruch, wobei jeder der Führungsschlitze ferner einen dritten Schlitzbereich in Kommunikation mit dem zweiten Schlitzbereich umfasst und ein Bogenmaß aufweist, das größer als das Bogenmaß des zweiten Schlitzbereichs ist, und wobei sich der zweite Schlitzbereich zwischen dem ersten Schlitzbereich und dem dritten Schlitzbereich befindet.

6. Herzklappenklemmvorrichtung nach Anspruch 1, wobei der Kupplungsmechanismus (3000) eine Betätigungsstange (3100) umfasst, die mit der Antriebsbaugruppe (2200) in einer nicht drehbaren und axial trennbaren Weise verbunden ist, sodass Drehung der Betätigungsstange (3100) die Antriebsbaugruppe (2200) anweist, sich axial zu bewegen; und die Betätigungsstange (3100) konfiguriert ist, um den Kupplungsmechanismus (3000) von der Befestigungsvorrichtung zu lösen, wenn die Betätigungsstange (3100) proximal bewegt wird.

7. Herzklappenklemmvorrichtung nach dem vorhergehenden Anspruch, wobei der Stützmechanismus (2000) eine feste Verbindungsbaugruppe (2100) umfasst und die Antriebsbaugruppe (2200) relativ zu der festen Verbindungsbaugruppe (2100) bewegbar ist, und wobei die feste Verbindungsbaugruppe (2100) ein Basiskupplungsende (2120) umfasst; wobei der Kupplungsmechanismus (3000) einen Kopplungssitz (3300), der trennbar mit dem Basiskupplungsende (2120) verbunden ist, und ein Eingriffselement (3200), welches das Basiskupplungsende (2120) und den Kopplungssitz (3300) verbindet, umfasst, und die Betätigungsstange (3100) eine Antriebskraft für die Antriebsbaugruppe (2200) bereitstellt; und die Betätigungsstange (3100) ein Betätigungsstangenkupplungsende (3120) umfasst, das mit der Antriebsbaugruppe (2200) auf eine axial trennbare und nicht drehbare Weise verbunden ist, wobei die Betätigungsstange (3100) konfiguriert ist, um relativ zu dem Eingriffselement (3200) zwischen einer ersten Position und einer zweiten Position bewegbar zu sein, und wenn die Betätigungsstange (3100) proximal von der zweiten Position bewegt wird, ein Betätigungsstangenstützabschnitt (3130) das Eingriffselement (3200) antreibt, um sich auch proximal zu bewegen, sodass der Kupplungssitz (3300) von dem Basiskupplungsende (2120) getrennt ist.

8. Herzklappenklemmvorrichtung nach dem vorhergehenden Anspruch, wobei der Kopplungssitz (3300) ein Kopplungssitzverbindungsende (3310), ein Kopplungssitzkupplungsende (3320) und einen Kopplungssitzinnenhohlraum (3330) definiert durch den Kopplungssitz (3300) umfasst, das Eingriffselement (3200) in dem Kopplungssitzinnenhohlraum (3330) bereitgestellt ist, das Kopplungssitzkupplungsende (3320) mit dem Basiskupplungsende (2120) über das Eingriffselement (3200) verbunden ist und das Kopplungssitzverbindungsende (3310) mit einer Abgabevorrichtung verbunden ist.

9. Herzklappenklemmvorrichtung nach dem vorhergehenden Anspruch, wobei die Abgabesteuervorrichtung ferner einen Manipulationsdraht (610) zum Steuern von Einfangelementen (1200) des Klemmmechanismus (1000) zum Öffnen und Schließen umfasst, und der Kopplungssitz (3300) eine Manipulationsdrahtbegrenzungsnut (3322) zum Einschränken von Trennung des Manipulationsdrahtes (610) umfasst, wenn der Kopplungssitz (3300) mit dem Basiskupplungsende (2120) verbunden ist.

10. Herzklappenklemmvorrichtung nach Anspruch 1, wobei die feste Verbindungsbaugruppe (2100) ein Basisgehäuse mit einem Basisinnenhohlraum (2130), der darin gebildet ist, einem Basisgewindeabschnitt (2131) in dem Basisinnenhohlraum (2130) umfasst, wobei die Antriebsbaugruppe (2200) eine Antriebswelle (2230) umfasst, wobei die Antriebswelle (2230) einen Gewindeabschnitt (2231) umfasst, der mit dem Basisgewindeabschnitt (2131) kooperiert, und wobei ein Steigungswinkel, bei dem der Basisgewindeabschnitt (2131) mit dem Antriebsabschnitt (2231) kooperiert, kleiner als ein Reibungswinkel ist.

## Revendications

1. Dispositif de pincement de valve cardiaque, comprenant :
un dispositif de fixation comprenant un mécanisme de pincement (1000) pour fermer une valve cardiaque, et un mécanisme de support (2000) portant le mécanisme de pincement (1000), le mécanisme de support (2000) comprenant un ensemble d'entraînement (2200) pour entraîner l'ouverture et la fermeture du mécanisme de pincement (1000) ; et
un dispositif de commande de distribution comprenant un arbre de poussée (600) pour introduire le dispositif de fixation à un emplacement cible et un mécanisme d'embrayage (3000) pour permettre à l'arbre de poussée (600) et au dispositif de fixation d'être reliés de manière séparable ; dans lequel
le mécanisme de pincement (1000) comprend une paire d'éléments de fermeture (1100) chacun doté d'une fente de guidage (1121) comprenant au moins un segment non linéaire, le mécanisme de support (2000) comprenant des éléments d'entraînement de fente (850), dont chacun est au moins partiellement situé dans les fentes de guidage (1121) et peut coulisser dans les fentes de guidage (1121), de façon à entraîner deux parties de pincement de fermeture (1110) pour qu'elles se rapprochent ou s'éloignent l'une de l'autre.

2. Dispositif de pincement de valve cardiaque selon la revendication précédente, dans lequel le mécanisme de pincement (1000) comprend la paire d'éléments de fermeture (1100) et une paire d'éléments de capture (1200), chaque élément de capture en correspondance un à un avec l'un respectif des éléments de fermeture (1100) ; la paire d'éléments de fermeture (1100) comprenant des parties de liaison de fermeture (1120) et les parties de pincement de fermeture (1110) en coopération avec la paire d'éléments de capture (1200) pour pincer la valve cardiaque ; et la fente de guidage (1121) formée dans les parties de liaison de fermeture (1120).

3. Dispositif de pincement de valve cardiaque selon la revendication précédente, dans lequel le mécanisme de support (2000) comprend un ensemble de liaison fixe (2100), les éléments d'entraînement de fente (850) dont chacun est au moins partiellement situé dans l'une des fentes de guidage (1121) sont prévus sur l'ensemble de liaison fixe (2100), et dans lequel l'ensemble d'entraînement (2200) est relié aux deux parties de pincement de fermeture (1120) et est agencé pour permettre à chacun des éléments d'entraînement de fente (850) de coulisser dans l'une respective des fentes de guidage (1121) lorsque l'ensemble d'entraînement (2200) se déplace par rapport à l'ensemble de liaison fixe (2100).

4. Dispositif de pincement de valve cardiaque selon l'une quelconque des revendications précédentes, dans lequel chacune des fentes de guidage (1121) comprend au moins deux régions de fente en communication l'une avec l'autre, et un radian d'une première région de fente des deux régions de fente est supérieur à un radian d'une deuxième région de fente des deux régions de fente.

5. Dispositif de pincement de valve cardiaque selon la revendication précédente, dans lequel chacune des fentes de guidage comprend en outre une troisième région de fente en communication avec la deuxième région de fente et présentant un radian supérieur au radian de la deuxième région de fente, et dans lequel la deuxième région de fente est située entre la première région de fente et la troisième région de fente.

6. Dispositif de pincement de valve cardiaque selon la revendication 1, dans lequel le mécanisme d'embrayage (3000) comprend une tige d'actionneur (3100) reliée à l'ensemble d'entraînement (2200) d'une manière non rotative et axialement séparable, de sorte qu'une rotation de la tige d'actionneur (3100) dirige l'ensemble d'entraînement (2200) pour qu'il se déplace axialement ; et la tige d'actionneur (3100) est conçue pour libérer le mécanisme d'embrayage (3000) du dispositif de fixation lorsque la tige d'actionneur (3100) est déplacée de manière proximale.

7. Dispositif de pincement de valve cardiaque selon la revendication précédente, dans lequel le mécanisme de support (2000) comprend un ensemble de liaison fixe (2100) et l'ensemble d'entraînement (2200) est mobile par rapport à l'ensemble de liaison fixe (2100), et dans lequel l'ensemble de liaison fixe (2100) comprend une extrémité d'embrayage de base (2120) ; le mécanisme d'embrayage (3000) comprend un siège d'accouplement (3300) relié de manière séparable à l'extrémité d'embrayage de base (2120) et un élément de prise (3200) reliant l'extrémité d'embrayage de base (2120) et le siège d'accouplement (3300), et la tige d'actionneur (3100) fournit une force d'entraînement pour l'ensemble d'entraînement (2200) ; et la tige d'actionneur (3100) comprend une extrémité d'embrayage de tige d'actionneur (3120) reliée à l'ensemble d'entraînement (2200) d'une manière axialement séparable et non rotative, la tige d'actionneur (3100) étant conçue pour être mobile par rapport à l'élément de prise (3200) entre une première position et une seconde position, et lorsque la tige d'actionneur (3100) est déplacée de manière proximale à partir de la seconde position, une partie de support de tige d'actionneur (3130) entraîne l'élément de prise (3200) pour qu'il se déplace également de manière proximale, de sorte que le siège d'accouplement (3300) est séparé de l'extrémité d'embrayage de base (2120).

8. Dispositif de pincement de valve cardiaque selon la revendication précédente, dans lequel le siège d'accouplement (3300) comprend une extrémité de liaison de siège d'accouplement (3310), une extrémité d'embrayage de siège d'accouplement (3320) et une cavité intérieure de siège d'accouplement (3330) définie à travers le siège d'accouplement (3300), l'élément de prise (3200) est prévu dans la cavité intérieure de siège d'accouplement (3330), l'extrémité d'embrayage de siège d'accouplement (3320) est reliée à l'extrémité d'embrayage de base (2120) par l'intermédiaire de l'élément de prise (3200), et l'extrémité de liaison de siège d'accouplement (3310) est reliée à un dispositif de distribution.

9. Dispositif de pincement de valve cardiaque selon la revendication précédente, dans lequel le dispositif de commande de distribution comprend en outre un fil de manipulation (610) pour commander l'ouverture et la fermeture des éléments de capture (1200) du mécanisme de pincement (1000), et le siège d'accouplement (3300) comprend une rainure de limitation de fil de manipulation (3322) pour restreindre une séparation du fil de manipulation (610) lorsque le siège d'accouplement (3300) est relié à l'extrémité d'embrayage de base (2120).

10. Dispositif de pincement de valve cardiaque selon la revendication 1, dans lequel l'ensemble de liaison fixe (2100) comprend un logement de base avec une cavité intérieure de base (2130) formée dans celui-ci, une partie filetée de base (2131) dans la cavité intérieure de base (2130), l'ensemble d'entraînement (2200) comprenant un arbre d'entraînement (2230), l'arbre d'entraînement (2230) comprenant une partie filetée (2231) coopérant avec la partie filetée de base (2131), et dans lequel un angle d'avance auquel la partie filetée de base (2131) coopère avec la partie d'entraînement (2231) est inférieur à un angle de frottement.
